# EUROPÄISCHE PATENTSCHRIFT

(11) **EP 0 667 131 B1**
(45) Veröffentlichungstag und Bekanntmachung des Hinweises auf die Patenterteilung: **03.09.2003**
(21) Anmeldenummer: 95100235.1
(22) Anmeldetag: 10.01.1995
(51) Int. Cl.: A61F 2/06

(54) **In den Körper eines Patienten perkutan implantierbare Endoprothese**
Percutaneous implantable endoprothesin in the body of a patient
Endoprothèse implantable percutanée dans le corps d'un patient

(30) Priorität: 18.01.1994 DE 4401227
(43) Veröffentlichungstag der Anmeldung: 16.08.1995
(73) Patentinhaber: Strecker, Ernst Peter, Dr.-med.Prof., D-76228 Karlsruhe (DE)
(72) Erfinder: Strecker, Ernst Peter, Dr.-med.Prof., D-76228 Karlsruhe (DE)
(74) Vertreter: Geitz, Holger

(56) Entgegenhaltungen:
- WO-A-91/19529
- WO-A-93/06792
- US-A- 4 423 725
- US-A- 4 922 905
- US-A- 5 156 620
- US-A- 5 232 444
- US-A- 5 242 397
- US-A- 5 312 339

## Beschreibung

Die Erfindung bezieht sich auf eine in den Körper eines Patienten, insbesondere in ein Blutgefäß oder eine andere Körperhöhle, mittels eines Katheters perkutan implantierbare Endoprothese, die als länglicher Hohlkörper ausgebildet und (nach lagerichtiger Plazierung bei der Implantation) von einem kleinen Lumen beim Einführen auf ein der Gebrauchslage entsprechendes größeres Lumen veränderbar ist.

Perkutan einführbare und im Lumen veränderbare Prothesen sind bekannt. Sie dienen zum Eröffnen oder Erweitern von Gefäßlumen entweder durch mechanisches Aufdehnen mittels bekannter Ballonkatheter von einem kleinen auf ein größeres Lumen oder sie dehnen sich nach vorheriger Zusammendrückung vor der Implantation durch Federkraft, bedingt durch beim Zusammendrücken erzeugte Federvorspannung, von selbst auf.

Eine auf einem Ballonkatheter aufgenommene und durch Dilatation aufweitbare sowie dadurch vom Katheter ablösbare und in einem Gefäß plazierbare Endoprothese ist in der EP-A-0 297 587 beschrieben. Bei dieser Prothese handelt es sich um einen durch Stricken, Häkeln oder sonstige Arten der Maschenbildung aus Metall- oder Kunststoff-Fadenmaterial guter Gewebeverträglichkeit hergestellten Stent in Form eines schlauchartigen Hohlkörpers, wobei die einzelnen Maschen aus locker ineinandergreifenden Schlingen bestehen. Beim Aufweiten infolge Dilatation des Ballons des Katheters erfahren die ineinandergreifenden Schlingen der Maschen plastische Verformungen und demgemäß verharrt eine aufgedehnte Prothese in ihrer Aufweitlage.

Selbstaufdehnende Stents sind beispielsweise in der EP-A-0 183 372 und in der US-PS 4 732 152 vorbeschrieben. Diese Prothesen werden vor der Implantation gegen ihnen eigene Federrückstellkräfte auf einen reduzierten Querschnitt zusammengedrückt, im zusammengedrückten Zustand in den Körper eines Patienten eingeführt und federn nach lagerichtiger Plazierung infolge Wegnahme der Rückhaltekraft im jeweiligen Gefäß oder Körperhohlraum auf und werden dadurch fixiert.

Bei der in der EP-A-0 183 372 beschriebenen Endoprothese handelt es sich darum, daß diese zum Zwecke der Implantation auf einen reduzierten Querschnitt zusammengedrückt und dann in diesem zusammengedrückten Zustand mittels eines sogenannten "Pushers" durch einen zuvor in ein Gefäß eingebrachten Katheter hindurch bis zur lagerichtigen Positionierung im Gefäß vorgeschoben wird. Dieses Vorschieben der Prothese durch den Katheter erfordert einen erheblichen Kraftaufwand, weil der Verschiebung große Reibungskräfte entgegenwirken.

Bei dem in der US-PS 4 732 152 beschriebenen System handelt es sich um eine gewebte und federnd ausgebildete Endoprothese, die im zusammengedrückten Zustand durch eine doppelte, am distalen Ende verschlossene Hülle zusammenghalten wird. Diese Hülle wid, wie beim Abstreifen eines Strumpfs vom Fuß eines Trägers, von der zusammengefalteten Prothese zurückgezogen. Zur Vermeidung der dabei auftretenden Reibung kann zwischen die beiden Hüllenblätter Flüssigkeit eingefüllt werden. Das wegen der Reduzierung der Reibungswiderstände zunächst elegant erscheinende System ist jedoch sehr umständlich in der Handhabung.

Aus der WO 93 121 985 A1 ist ein Ballonkatheter mit einem konstanten Lumen zur Durchführung einer Angioplastie bekannt, der von einem Medikamentengabeballon zur Behandlung der Gefäßwände umschlossen ist. Der Ballon des Ballonkatheters schließt mit der porösen Wandung des Medikamentengabeballons einen Zwischenraum ein, in den durch einen Zuführschlauch Medikamente einbringbar sind. Die jeweilige Dosierung der durch die Wandung des Medikamentengabeballons dringenden Substanzen ist stark vom Innendruck des Ballonkatheters abhängig. Eine starke Ballonaufweitung zur Durchführung der Angioplastie hat unabhängig von der therapeutischen Notwendigkeit eine stärkere Medikamentengabe zur Folge. Wenn der Medikamentengabeballon direkt an der Gefäßwand anliegt, können die mit Druck austretenden Medikamente die Gefäßwandungen verletzen. Eine von der jeweiligen Ballonaufweitung unabhängige Medikamentengabe ist bei dieser Vorrichtung nicht möglich.

Die der Erfindung zugrundeliegende Aufgabe besteht demgegenüber darin, die eingangs erläuterte gattungsgemäße Endoprothese, die in ihrem Lumen veränderbar und mittels eines Katheters implantierbar ist, dahingehend zu verbessern, daß diese neben ihrer Funktion, eine Verbindung zu oder zwischen Körperhohlräumen herzustellen und nach der Implantation auf Dauer offen zu halten, auch für therapeutische Zwecke brauchbar ist.

Diese Aufgabe ist bei der Endoprothese nach dem Oberbegriff des Patentanspruchs 1 dadurch gelöst, daß ein Stützkörper der Endoprothese zumindest außenseitig von einer zusammen mit dem Stützkörper von einem kleineren Einführlumen auf ein größeres Implantations-Lumen aufweitbaren Manschette umschlossen ist und daß wenigstens ein mit dieser Manschette verbundener Zuführschlauch zum Zuführen von Medikamenten oder anderen Substanzen in einen sich im eingeführten Zustand zwischen der Manschette und der die Manschette umschließenden Körperhöhle ausbildenden Zwischenraum ausmündet.

Bei der Erfindung handelt es sich somit um eine Endoprothese, die im eingeführten Zustand mit der den Stützkörper zumindest außenseitig umschließenden Manschette sich an die einen Implantationsraum begrenzenden Wandungen, beispielsweise an die Wandungen eines die Endoprothese aufnehmenden Blutgefäßes, anlegt und dann die Zufuhr von Medikamenten oder sonstigen Substanzen in den Zwischenraum zwischen den genannten Wandungen und der Manschette ermöglicht. Auf diese Weise können Medikamentendepots angelegt werden, die eine gezielte therapeutische Behandlung der umgebenden Wandungsbereiche ermöglichen, indem die Medikamente in die umgebenden Wandungen eindringen.

Zweckmäßigerweise kann der Zuführschlauch an einem Stirnende der Manschette oder in der Nähe dieses Stirnendes ausmünden. Die Manschette kann aus plastisch aufdehnbarem, aber auch aus elastischem Material mit einer begrenzten Rückstellkraft bestehen.

Nach Anspruch 4 weist die Manschette gegenüber dem Stützkörper ein Übermaß auf und ist unter Ausbildung von Falten um den Stützkörper herumgelegt, die sich beim Aufdehnen des Stützkörpers straffen.

Obgleich Versuche gezeigt haben, daß bei Endoprothesen der vorstehenden Gestaltung im stirnendigen Bereich der Endoprothese eine hinreichende Abdichtung gegen das unerwünschte Abwandern der über den Zuführschlauch zugeführten Medikamente gewährleistet ist, hat sich eine Ausbildung der Endoprothese derart als zweckmäßig erwiesen, daß die Stirnenden der Endoprothese im aufgeweiteten Zustand Übermaß gegenüber dem Mittelteil der Endoprothese haben.

Gemäß Anspruch 6 ist die Endoprothese an ihren Stirnenden mit radial nach außen vorspringenden Wulsten versehen, so daß im Bereich zwischen diesen Wulsten zwischen der Endoprothese und den Körpergefäßwandungen eine Infusionskammer gebildet ist, in die mittels des Zuführschlauches Medikamente oder andere Substanzen eingebracht werden können. Falls die Wulste nicht mit der Gefäßwandung abschließen, entsteht eine leckende Infusionskammer. In dieser Kammer ist dennoch eine immerhin erhöhte Konzentration der eingebrachten Substanzen gegeben. Gleichzeitig ist weiterhin eine Blutdurchströmung dieser Kammer möglich.

Bei einer anderen Weiterbildung der Erfindung schließen die Wulste mit den Gefäßwandungen dichtend ab. Bei dieser Weiterbildung ist es vorteilhaft, daß das Abströmen der zugeführten Substanzen mit dem Blutstrom wirksam verhindert ist. Dadurch, daß die Wulste elastisch sind, ist ein gutes Anliegen an den umgebenden Gefäßwandungen gewährleistet.

Nach Anspruch 8 sind die Wulste der Endoprothese verbreitert. Hierdurch ist die an der Gefäßinnenwand anliegende Fläche der Wulste vergrößert. Die Dichtwirkung, die durch die Wulste erzielt wird, ist somit ebenfalls vergrößert.

Eine abermalige Weiterbildung der Erfindung sieht vor, daß beabstandet von den Stirnenden der Manschette diese mit außenseitig radial vorstehenden Distanzmitteln versehen ist, die eine vorbestimmte Distanzhaltung gegenüber umgebenden Wandungen des die Endoprothese aufnehmenden Körperhohlraums gewährleisten und damit eine entsprechend voluminöse Ausbildung des Zwischenraums zwischen diesen Körperwandungen und der Manschette sicherstellen.

Eine gemäß Anspruch 10 ausgestaltete Endoprothese kann vorteilhaft dadurch entfernt werden, daß zunächst der Stützkörper der Endoprothese mit dem ersten Hilfskatheter zurückgezogen und entfernt wird. Bei Verwendung eines gestrickten Drahtstützkörpers wird hierbei durch Zug der Radius des Stützkörpers verkleinert. Anschließend wird die Manschette mittels des zweiten Hilfskatheters zurückgezogen und ebenfalls entfernt.

Nach Anspruch 11 ist die Manschette mit einer außenseitigen Antihaftbeschichtung, insbesondere aus Silikonöl, versehen. Hierdurch ist sichergestellt, daß der zur Infusion von Medikamenten benutzte Zwischenraum offengehalten ist und die Ausmündungen der Manschette für die Zufuhr von Medikamenten oder anderen Substanzen nicht etwa verschlossen sind.

Dadurch, daß gemäß Anspruch 12 die Manschette als Hohlmembran ausgebildet ist, können Medikamentendepots innerhalb des zu behandelnden Gefäßes angelegt werden.

In Verbindung mit aufdehnbaren Stützkörpern hat es sich als besonders vorteilhaft erwiesen, wenn auch die Manschette aus elastisch aufdehnbarem Material hergestellt ist. Dies ist insbesondere für die Behandlung einer Aneurysma ein wichtiges Merkmal. Es handelt sich dabei um eine krankhafte örtlich begrenzte Erweiterung eines Blutgefäßes.

Anstelle von Medikamenten können aber auch in dem sich bei der Implantation ausbildenden Zwischenraum zwischen der Manschette und den Wandungen des die Endoprothese aufnehmenden Körperhohlraums andere Substanzen zugeführt werden. Insbesondere kann es sich dabei auch um aushärtende Substanzen handeln, die ihrerseits nach dem Aushärten dann eine tunnelartige Abstützung der umgebenden Körper- oder Gefäßwandungen vermitteln und sich lediglich mit der Gefäßwand verbinden. Hierdurch wird das nachträgliche Entfernen der Endoprothese erleichtert. Diese Substanzen können auch mit eingelagerten Medikamenten versehen und/ oder biologisch abbaubar sein.

Um eine Versorgung beispielsweise umgebender Gefäßwandungen durch den im Gefäß geführten Blutstrom zu ermöglichen, sollten derartige Tunnel aus aushärtender Masse mit sich durchgängig bis zu den Gefäßwandungen durch die Tunnelwandungen hindurcherstreckenden Lochungen bzw. Poren versehen sein. Dies ist in einfacher Weise verwirklichbar, wenn die Manschette mit entsprechend radial vorstehenden Distanzmitteln oder einer entsprechenden außenseitigen Profilierung versehen ist.

Bei der zuletzt genannten Zweckbestimmung der Endoprothese hat sich auch als vorteilhaft erwiesen, wenn die Manschette außenseitig mit einer Antihaftbeschichtung versehen ist, die nach dem Aushärten der in den Zwischenraum zwischen der Manschette und den umgebenden Gefäßwandungen eingefüllten Substanzen ein leichtes Ablösen der Manschette von dieser ausgehärteten Substanz ermöglicht. Als vorteilhafte und einfach aufbringbare Antihaftbeschichtung hat sich ein Auftrag einer Silikonschicht auf die Außenfläche der Manschette erwiesen.

Ebenfalls im Rahmen der Erfindung kann aber auch die den Stützkörper der Endoprothese umschließende Manschette als schlauchartige Hohlmembran mit mindestens einem im Manschettenhohlraum ausmündenden Zuführschlauch zum Zuführen von Medikamenten oder anderen Substanzen ausgebildet sein. Wenn diese Hohlmembran mit Lochungen vorbestimmter Größe oder einer gewissen Porosität ausgerüstet ist, gelingt in hervorragender Weise eine allmähliche Medikamentenabgabe.

Eine Endoprothese mit einer als schlauchartige Hohlmembran ausgebildeten Manschette aus elastisch aufdehnbarem Material kann aber auch insbesondere dazu dienen, Aneurysmen an Gefäßen zu verschließen, indem eine auf einem Katheter aufgenommene Endoprothese mit einer derartigen Hohlmembran gezielt in den Bereich einer Gefäßausbuchtung gebracht und dann letztere durch Aufdehnen der Hohlmembran ausgefüllt wird, indem über die Zuführleitung ein das Aufdehnen der Hohlmembran bewirkendes und danach aushärtendes Mittel in den Membranhohlraum eingebracht wird.

Um auch extrem ausgebildete Aneurysmen an Gefäßen verschließen zu können, sieht eine nochmalige Weiterbildung der Erfindung gemäß Anspruch 13 vor, daß die Hohlmembran Übermaß gegenüber dem Stützkörper der Prothese aufweist, mit diesem zumindest partiell verbunden und im übrigen unter Faltenbildung um den Stützkörper herumgelegt ist. Wenn in eine so ausgebildete Hohlmembran über die genannte Zuführleitung ein Füllmittel eingebracht wird, faltet sich die Prothese nach und nach auf und füllt auch größere Gefäßausbuchtungen oder Gefäßsäcke gezielt aus. Als besonders zweckmäßig hat sich dabei erwiesen, wenn die als Hohlmembran ausgebildete Manschette von deren Stirnenden aus longitudinal eingefaltet ist.

Gemäß einer anderen Weiterbildung kann der Zuführschlauch lösbar mit der Manschette verbunden und mithin nach einer den therapeutischen Zwecken genügenden Medikamentenzufuhr oder nach der Zufuhr einer abbindenden oder erhärtenden Substanz von der Hohlmembran abgetrennt bzw. aus dieser herausgezogen werden, wobei dann wiederum eine die Abdichtung der Hohlmembran vermittelnde Einrichtung vorgesehen sein muß, beispielsweise durch ein geeignetes Ventil.

Eine konkrete Ausgestaltung einer derart wieder verschließbaren Ausmündung einer Manschette ist durch eine Endoprothese nach Anspruch 16 oder 17 gegeben.

Sieht man wenigstens zwei Zuführschläuche zur Medikamentengabe oder Substanzenzufuhr vor, so können dem Zwischenraum auch Zweikomponentenreaktionsgemische zugeführt werden, die erst in dem Zwischenraum miteinander zu der bestimmungsgemäßen Substanz reagieren, wie beispielsweise Epoxidharze oder Gewebekleber oder Kleber, die durch Anwesenheit von Ionen aushärten.

Als gleichfalls vorteilhaft hat sich erwiesen, wenn die den Stützkörper umschließende Manschette aus biologisch abbaubarem Material besteht, so daß im Verlaufe eines vorbestimmten Zeitraums die eingebrachte Manschette sich im Körper eines Patienten auflöst.

Bei der erfindungsgemäßen Endoprothese können Stützkörper unterschiedlichster Art eingesetzt werden. Als besonders vorteilhaft hat sich indessen erwiesen, wenn der von der Manschette umschlossene Stützkörper als durch Stricken oder ähnliche Formen der Maschenbildung hergestelltes schlauchartiges Maschenwerk ausgebildet ist. Ein derartiges Maschenwerk zeichnet sich durch eine gewisse Elastizität im aufgeweiteten Zustand aus, die selbst nach äußeren Einwirkungen und dadurch bedingten leichten Deformationen ein Auffedern in den aufgeweiteten Zustand gewährleistet. Diese Stützkörper können selbstaufdehnend ausgebildet oder mittels Ballonkatheter aufweitbar sein, aber auch aus unter dem Einfluß der Körpertemperatur im implantierten Zustand in die Aufweitlage gehendem Memorymaterial bestehen.

Bei einer derartigen Stützkörperausbildung kann sich vom proximalen Stirnende des als Maschenwerk ausgebildeten Stützkörpers auch ein das Aufziehen des Maschenwerks ermöglichendes Endfilament forterstrecken. Mittels dieses Endfilaments kann das Maschenwerk aufgezogen und dadurch der Stützkörper in einfacher Weise nach dem Einführen wieder entfernt werden.

Das Endfilament kann auch mit dem sich ebenfalls von der proximalen Stirnseite forterstreckenden Zuführungsschlauch fest verbunden sein, so daß ein gleichzeitiges Entfernen des Stützkörpers und der diesen umgebenden Manschette möglich ist. Durch axialen Zug an der Prothese reduziert sich der Querschnitt des Stützkörpers, so daß ein unproblematisches Entfernen der Endoprothese beispielsweise mittels eines Katheters möglich ist.

Gemäß einer abermaligen Weiterbildung nach Anspruch 22 kann nach dem Aufziehen des Maschenwerks die Manschette an dem Endfilament aus dem Implantationsraum zurückgezogen werden.

Vorteilhafterweise ist der Stützkörper der Endoprothese aus einem isolierten elektrisch leitenden Thermo-Memory-Draht hergestellt, der sich unter Wärmeeinwirkung so zusammenzieht, daß der gesamte Stützkörper in seinem radialen Querschnitt verkleinert ist. Hierdurch ist das Entfernen der Endoprothese nach der Behandlung erleichtert. Optimalerweise reagiert der Thermo-Memory-Draht bei einer Temperatur, die nur wenig über der üblichen Bluttemperatur liegt. Dadurch werden Schädigungen des umliegenden Gewebes des zu behandelnden Körpergefäßes vermieden.

Zur Erzeugung der leicht erhöhten Temperatur wird bei einer Endoprothese nach Anspruch 24 eine leicht erwärmte Flüssigkeit über einen Zusatzkatheter in Strömungsrichtung vor der Endoprothese zugeführt, so daß die Endoprothese von dieser erwärmten Flüssigkeit unter Wärmeabgabe durchströmt wird.

Nach Anspruch 25 kann der Stützkörper der Endoprothese auch dadurch erwärmt werden, daß ein elektrischer Strom durch den isolierten Thermo-Memory-Draht fließt.

Bei einer Ausgestaltung der Endoprothese nach Anspruch 26 wird nur ein Teil der Endoprothese aus einem Katheter in ein Körpergefäß geschoben. In den meisten Fällen handelt es sich hierbei um eine selbstaufdehnende Endoprothese. Der in das Körpergefäß geschobene Teil der Endoprothese weist ebenfalls radial vorstehende Wulste auf, die einen Zwischenraum begrenzen. Der proximalseitige Wulst wird derart von der Manschette der Endoprothese übergriffen, daß die Manschette sich konisch verjüngend ebenfalls in dem Katheter ausläuft. Dieser zwischen dem proximalseitigen Wulst der Endoprothese und dem Katheter angeordnete Konus weist Seitlöcher auf. Durch diese Seitlöcher kann eine nahezu ungehinderte Blutperfusion durch das zu behandelnde Körpergefäß und die Endoprothese erfolgen.

Die Endoprothese kann in Weiterbildung dieser Ausgestaltung durch einen im Katheter angeordneten Pusher problemlos positioniert und verschoben werden. An diesem Pusher kann die Endoprothese durch den Katheter wieder aus dem Körpergefäß herausgezogen werden.

Eine weiter verbesserte Blutdurchströmung der Endoprothese ist dadurch möglich, daß der Katheter exzentrisch zu dem aufgedehnten Teil der Endoprothese angeordnet ist und der somit ebenfalls exzentrisch verlaufende Konus der Manschette an seiner entsprechend vergrößerten Konusfläche mit wenigstens einem vergrößerten Seitloch versehen ist. Der hierdurch verbesserte Blutdurchfluß reduziert die Gefahr der Thrombenbildung in dem neu zu behandelnden Körpergefäß.

In vorteilhafter Weiterbildung dieser Ausgestaltung besteht der Stützkörper dieser Endoprothese aus hoch- bis superelastischem Draht, wie beispielsweise Nitinaldraht. Diese Ausgestaltung der endoprothese kann nach Anspruch 30 auch in Verbindung mit ballonaufdehnbaren Stützkörpern eingesetzt werden.

Eine andere Ausgestaltung der Endoprothese ist nach Anspruch 31 gegeben. Bei dieser Ausgestaltung ist nach dem Einführen der Endoprothese der Zwischenraum zwischen Endoprothese und Gefäßwand mit einem Schwamm, schwammartigen Gel oder einer anderen schwammartigen Masse angefüllt. Dieser Schwammbereich wird über den Zuführschlauch mit einer medikamentösen oder anderen Substanz getränkt.

Ein Stoffwechsel mit der Gefäßwand findet dann über den Schwammbereich statt. Nach der Behandlung des Gefäßes wird die Endoprothese mitsamt der den Zwischenraum ausfüllenden Masse entfernt.

Anhand der beigefügten Zeichnung sollen nachstehend verschiedene Ausführungsformen der erfindungsgemäßen Endoprothese erläutert werden. In schematischen Ansichten zeigen:
- Fig. 1: einen Längsschnitt durch eine eingeführte Endoprothese, die als länglicher Hohlkörper mit einem inneren Stützkörper in Form eines durch Stricken hergestellten schlauchartigen Maschenwerks sowie mit einer letzteres umschließenden Manschette ausgebildet ist und mit ihren stärker als das Mittelteil aufgeweiteten Enden dichtend an der Wandung eines nur angedeuteten Blutgefäßes anliegt,
- Fig. 2: in einer Ausschnittansicht den Aufbau des Stützkörpers als aus metallischem Fadenmaterial hergestelltes Gestricke mit locker ineinandergreifenden und maschenbildenden Schlingen,
- Fig. 3: in einer Ansicht wie in Fig. 1 eine abgewandelte Prothesenausführung, bei der eine den Stützkörper umschließende Manschette an ihren Enden mit radial über den Manschettenmantel vorstehenden Kragen versehen ist,
- Fig. 4: die Endoprothese nach Fig. 3 im nicht aufgeweiteten Zustand, jedoch mit von der den Stützkörper umgebenden Manschette noppenartig vorstehenden Abstandshaltern,
- Fig. 5: einen Längsschnitt durch eine Endoprothese mit einer Hohlmembran als den Stützkörper umgebende Manschette,
- Fig. 6: eine Längsschnittansicht wie in den Fig. 1 und 3 die eingeführte Endoprothese nach Fig. 5 mit aufgedehnter und eine umlaufende Gefäßausbuchtung ausfüllender Hohlmembran,
- Fig. 7: einen Längsschnitt durch eine eingeführte Endoprothese und einen Hauptkatheter mit zwei Hilfskathetern,
- Fig. 8: einen Längsschnitt durch eine eingeführte Endoprothese mit einem Stützkörper aus elektrisch leitenden Thermo-Memory-Draht und
- Fig. 9: einen Längsschnitt durch eine eingeführte Endoprothese, die nur zum Teil aus einem Hauptkatheter geschoben ist.

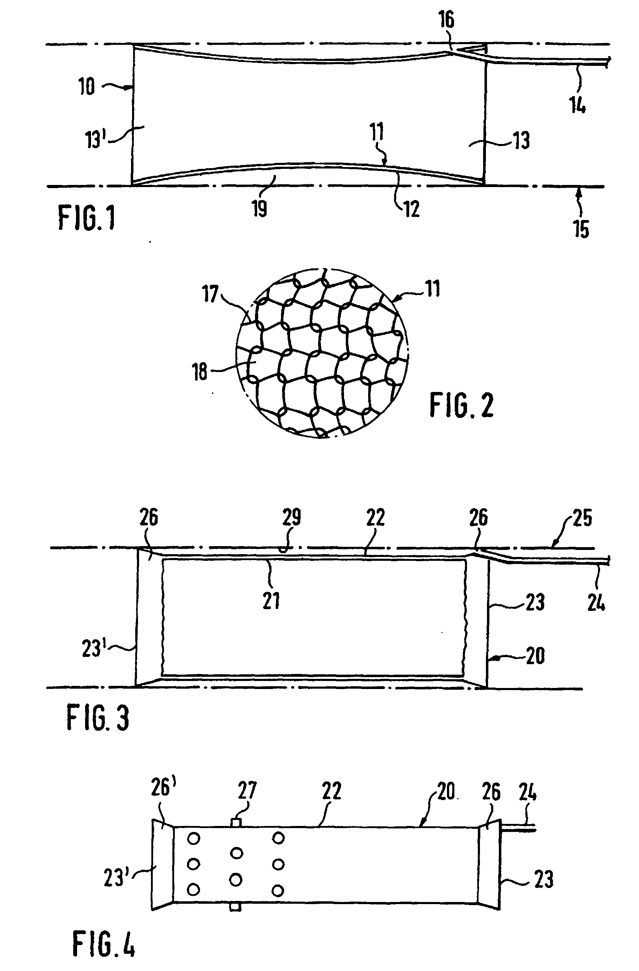

Bei der in Fig. 1 im eingeführten Zustand gezeigten Endoprothese 10 handelt es sich um einen sich selbst aufdehnenden Stent in Form eines langgestreckten Hohlkörpers mit einem als schlauchartiges Gestricke ausgebildeten inneren Stützkörper 11 und einer letzteren außenseitig umschließenden Manschette 12. Die Manschette 12 ist zumindest partiell fest mit dem Stützkörper 11 verbunden und besteht aus elastischem Material, das problemlos aufdehnbar ist. In der Nähe eines proximalen Stirnendes 13 der Manschette 12 ist an diese ein Zuführschlauch 14 angeschlossen, der sich axial von der Endoprothese 10 im Lumen eine die Endoprothese 10 aufnehmenden Körpergefäßes 15 forterstreckt und in einer die Manschette durchbrechenden Ausmündung 16 endet.

Fig. 2 veranschaulicht in einem Wandausschnitt aus einem als schlauchartiges Gestricke aus metallischem Fadenmaterial 17 hergestellten schlauchartigen Stützkörper 11 der Endoprothese 10, bei der locker ineinandergreifende Schlingen offene Maschen 18 bilden. Der besondere Vorteil einer derartigen Stützkörperausbildung besteht darin, daß die Endoprothese 10 elastisch nachgiebig ist und nicht nur Gefäßkrümmungen bei der Implantation zu folgen vermag, sondern im implantierten Zustand infolge äußerer Krafteinwirkungen auftretende Deformationen werden von einem . derartigen Stützkörper 11 angesichts der diesem eigenen Federrückstellkräfte wieder ausgeglichen.

Bei der Endoprothese 10 nach Fig. 1 ist der Stützkörper 11 tailliert. Demgemäß haben die beiden Stirnenden 13, 13' der Endoprothese 10 Übermaß gegenüber einem zwischen diesen Stirnenden 13, 13' befindlichen Mittelteil. Dies vermittelt im implantierten Zustand ein dichtes Anliegen der Endoprothese 10 im Bereich der Stirnenden 13, 13' an den strichpunktiert in Fig. 1 angedeuteten Wänden eines die Endoprothese 10 aufnehmenden Körpergefäßes 15. Dadurch bildet sich zwischen der Gefäßwand und der den Stützkörper außenseitig umschließenden Manschette 12 ein umlaufender Zwischenraum 19, in die über den Zuführschlauch 14 ein Medikament einführbar ist, das nach und nach in die Gefäßwand eindringt.

Der in einer Ausmündung 16 die Manschette 12 in der Nähe des proximalen Stirnendes 13 durchdringende Zuführschlauch 14 kann auch lösbar mit der Manschette 12 verbunden sein, so daß nach erfolgter Medikamentenzufuhr der Zuführschlauch 14 von der Manschette 12 entfernbar ist. Zweckmäßigerweise ist im Bereich der Ausmündung 16 eine nicht dargestellte Absperreinrichtung vorgesehen, die beim Entfernen des Zuführschlauchs 14 die Ausmündung 16 abschließt und dadurch ein Abfließen des in den kreisringförmigen Zwischenraum 19 aufgenommenen Medikaments in das Gefäßlumen verhindert.

Implantiert wird die Endoprothese 10 bei reduziertem Querschnitt mittels eines Katheters. Zu diesem Zwecke wird die Endoprothese 10 radial zusammengedrückt und mittels einer nach dem proximalen Stirnende 13 hin abziehbaren Umhüllung auf dem Katheter gehalten. Nach dem lagerichtigen Einführen des Katheters mit der darauf aufgenommenen Endoprothese 10 in ein Körpergefäß 15 wird die die Endoprothese 10 in zusammengedrückter Lage haltende Hülse abgezogen und die Endoprothese 10 erfährt angesichts der dem Stützkörper 11 infolge der vorherigen Zusammendrückung aufgeprägten Federvorspannung eine radiale Aufweitung, wobei angesichts der taillierten Ausbildung die Endoprothese 10 an den Stirnenden 13, 13' zur dichtenden Anlage an der Gefäßwand gelangen.

Die in Fig. 3 ebenfalls im implantierten Zustand veranschaulichte Endoprothese 20 unterscheidet sich von der Endoprothese 10 nach Fig. 1 dadurch, daß die den inneren Stützkörper 21 umschließende Manschette 22 beidendig mit radial vorstehenden Wulsten 23, 23', die in Form von kragenartigen Endabschnitten ausgebildet sind, versehen ist, die axial über den Stützkörper 21 hinausragen und im implantierten Zustande dichtend an der Wand eines die Endoprothese 10 aufnehmenden Körpergefäßes 25 anliegen. Diese Wulste 23, 23' haben die Form konischer Erweiterungen. Im Bereich des proximalen Wulstes 23 mündet wiederum ein Zuführschlauch 24 aus, der sich in gleicher Weise wie bei der Ausführungsform nach Fig. 1 im Lumen des die Endoprothese 10 aufnehmenden Blutgefäßes 15 forterstreckt und die Zufuhr von Medikamenten oder anderen Substanzen in den zwischen der Gefäßwand und der Manschette 22 ausgebildeten Zwischenraum 29 ermöglicht.

Anstelle eines Medikaments kann in den kreisringförmigen Zwischenraum 29 zwischen der Gefäßwand und der den inneren Stützkörper 21 umschließenden Manschette 22 auch eine aushärtende Substanz eingefüllt werden, die nach dem Aushärten eine tunnelartige Gefäßabstützung bildet. Nach dem Aushärten dieser Masse ist die Endoprothese 10 entfernbar, indem beispielsweise ein Endfilament vom proximalen Ende des Stützkörpers 21 vorsteht und ebenso wie der Zuführschlauch 24 durch das die Endoprothese 20 aufnehmende Lumen und eine Punktionsstelle hindurchgeführt ist, mittels dessen die Maschen des Stützkörpers 21 aufziehbar sind. Die Manschette 22 ist nach dem Aufziehen der Maschen des Stützkörpers 21 in einfacher Weise mittels des Zuführschlauchs 24 von der Implantationsstelle zurückziehbar. Es kann aber auch ein Endfilament des Stützkörpers 24 im Bereich des distalen Stirnendes 23' mit der Manschette 22 verbunden sein, so daß im Anschluß an das Aufziehen der Maschen des Stützkörpers 21 die Manschette 22 über dieses Endfilament zurückziehbar ist.

Beim Verfüllen des Zwischenraums 29 zwischen der Gefäßwand und der Manschette 22 der Endoprothese 20 mit einer aushärtbaren Masse hat sich als zweckmäßig erwiesen, wenn die den Stützkörper 21 umschließende Manschette 22 mit Distanzmitteln (27) in Form von außenseitig vorstehenden noppenartigen Vorsprüngen ausgerüstet ist, wie dies Fig. 4 zeigt. Diese Noppen wirken als Abstandshalter und legen sich im implantierten Zustand an die Wand des die Endoprothese 20 aufnehmenden Körpergefäßes 25 an.

Anstelle derartiger Noppen kann die Manschette 22 auch mit einer außenseitigen Profilierung versehen sein. Bei einer so ausgebildeten Endoprothese 20 weist nach dem Aushärten der in den Zwischenraum 29 zwischen der Manschette 22 und der Gefäßwand eingefüllten Masse die tunnelartige Abstützung eine Vielzahl von Durchbrechungen oder Lochungen auf, die eine Ernährung der Gefäßwand durch den im Körpergefäß 25 fließenden Blutstrom sicherstellen.

Bei Endoprothesen 20, die für das Herstellen tunnelartiger Abstützungen in einem Körpergefäß 25 mittels einer aushärtbaren Substanz bestimmt sind, hat sich auch als vorteilhaft erwiesen, wenn die Manschette 22 außenseitig mit einer Antihaftbeschichtung versehen ist. Bei dieser Antihaftbeschichtung kann es sich beispielsweise um einen dünnschichtigen Auftrag aus Silikonöl handeln.

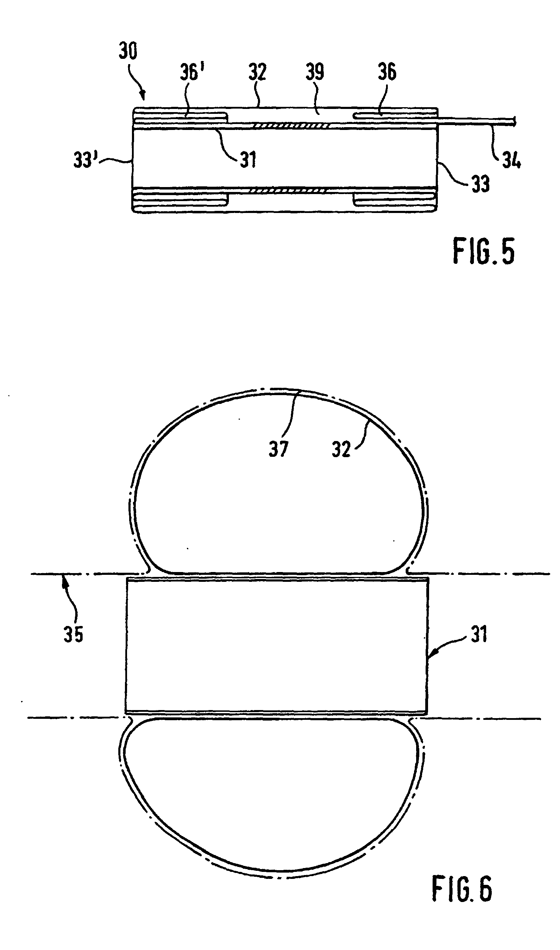

Bei der in Fig. 5 veranschaulichten Endoprothese 30 ist auf einem Stützkörper 31 die Manschette 32 in Form eines schlauchartigen Gestrickes als Hohlmembran aufgenommen, die mit dem Stützkörper 31 nur im mittleren Bereich verbunden ist. Diese Manschette 32, an die ebenfalls ein Zuführschlauch 34 angeschlossen ist, ist in der Art eines Faltenbalgs mit stirnendigen Longitudinalfalten 36, 36' ausgerüstet. Der Zuführschlauch 34 erstreckt sich vom proximalen Ende fort.

Die Endoprothese 30 gemäß Fig. 5 ist insbesondere zum dauerhaften Verfüllen von Aneurysmen bestimmt. Dies zeigt Fig. 6. Bei derartigen Aneurysmen handelt es sich um Gefäßausbuchtungen 37, in deren Bereich es zu gefährlichen Blutungen kommen kann.

Zur Behandlung derartiger Gefäßausbuchtungen 37 wird die Endoprothese 30 nach Fig. 5 mittels eines nicht dargestellten Katheters lagerichtig in ein Körpergefäß 35 eingeführt, wie dies Fig. 6 zeigt. Nach der Freigabe der Endoprothese 30 dehnt sich diese bis zum Anliegen an der Gefäßwand auf. Danach wird die als Hohlmembran ausgebildete Manschette 32 über den Zuführschlauch 34 mit einer aushärtbaren Substanz verfüllt mit der Folge, daß die Hohlmanschette 32 sich in die das Gefäß 35 zirkulär umgebende Gefäßausbuchtung 37 hineinverformt und diese vollständig ausfüllt. Nach dem Aushärten der in den Innenraum der Hohlmembran verfüllten Masse wird der Zuführschlauch 34 zurückgezogen und erforderlichenfalls der Stützkörper 31 der Endoprothese durch Aufziehen der Maschen des Stützkörpers 31 entfernt. Die nur im Mittelteil der Endoprothese 30 mit der Manschette 32 beispielsweise durch Klebung verbundenen Filamente des Stützkörpers 31 lösen sich beim Aufziehen der Maschen von der Manschette 32.

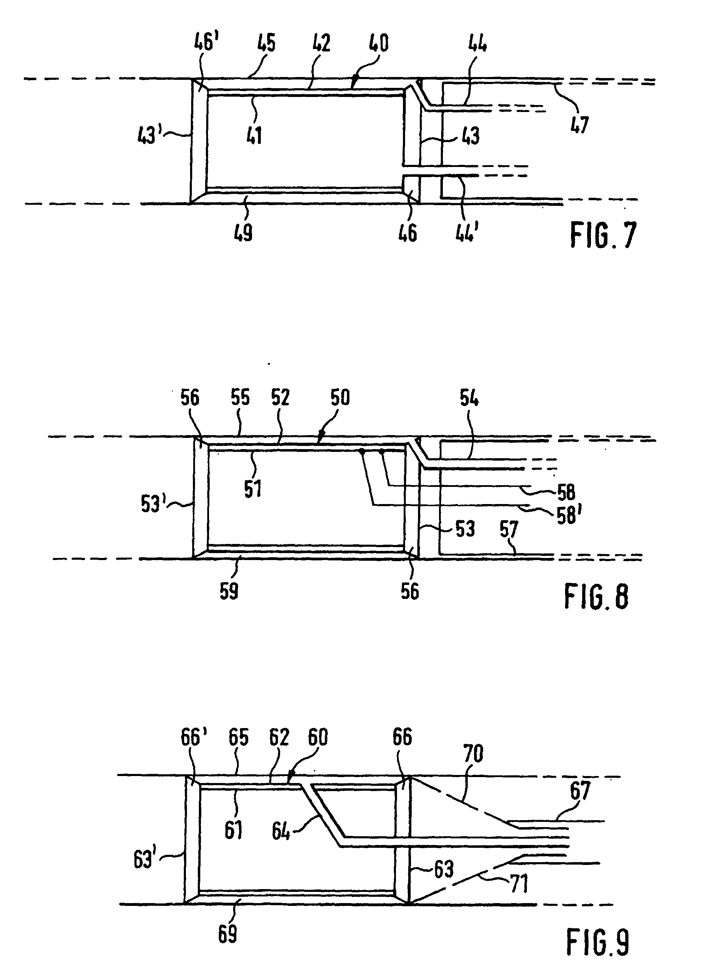

In den Fig. 7, 8 und 9 sind jeweils in ein Körpergefäß 45, 55, 65 implantierte Endoprothesen 40, 50, 60 grob vereinfacht dargestellt. Die Endoprothesen 40, 50, 60 sind jeweils mittels eines Hauptkatheters 47, 57, 67 in das Körpergefäß 45, 55, 65 eingebracht worden. Der Stützkörper 41, 51, 61 der Endoprothesen 40, 50, 60 ist jeweils von einer Manschette 42, 52, 62 umschlossen. An den Stirnenden 43, 43' bzw. 53, 53' bzw. 63, 63' sind jeweils radial vorstehende Wulste 46, 46' bzw. 56, 56' bzw. 66, 66' angeordnet, die jeweils einen zwischen der Gefäßinnenwandung und der Manschette 42, 52, 62 gebildeten Zwischenraum 49, 59, 69 abschließen.

Die Manschette 42 der in Fig. 7 gezeigten Endoprothese 40 ist mit einem ersten Hilfskatheter 44 und der Stützkörper 41 dieser Endoprothese 40 mit einem zweiten Hilfskatheter 44' verbunden. Die Endoprothese 40 kann nach der Behandlung des Körpergefäßes 45 dadurch entfernt werden, daß zunächst an dem zweiten Hilfskatheter 44' gezogen wird. Durch eine derart auf den Stützkörper 41 aufgebrachte Zugkraft verjüngt sich dessen Querschnitt und der Stützkörper 41 kann durch den Hauptkatheter 47 aus dem Körpergefäß 45 herausgezogen werden. Anschließend wird an dem ersten Hilfskatheter 44 die inzwischen zusammengefallene Manschette 42 aus dem Körpergefäß 45 gezogen.

Der Stützkörper 51 der in Fig. 8 dargestellten Endoprothese 50 ist ein durch Stricken herstellbares Maschengeflecht aus einem elektrisch leitenden Thermo-Memory-Draht. Dieser Stützkörper 51 ist so mit einer Stromzuführungsleitung 58 und einer Stromabführungsleitung 58' verbunden, daß möglichst das gesamte Maschengeflecht des Stützkörpers 51 strombeaufschlagbar ist. Die beiden Stromleitungen 58, 58' sind gegeneinander isoliert entlang des Zuführschlauches 54 im Hauptkatheter 57 verlegt. Die Endoprothese 50 kann vorteilhaft dadurch entfernt werden, daß der Stützkörper 51 derart strombeaufschlagt wird, daß sich die Temperatur des Stützkörpers 51 leicht über die normale Bluttemperatur erhöht. Das umliegende Gewebe wird durch diesen geringen Temperaturanstieg nicht beeinträchtigt. Der Thermo-Memory-Draht zieht sich infolge dieses Temperaturanstieges zusammen, so daß der hierdurch in seinem Querschnitt verkleinerte Stützkörper durch den Hauptkatheter 57 entfernt werden kann.

Die in Fig. 9 dargestellte Endoprothese 60 ist nur zum Teil in das Körpergefäß 65 eingebracht. Der andere Teil der Endoprothese 60 verbleibt im Hauptkatheter 67. Die Endoprothese 60 ist selbstaufdehnend, so daß der jeweils aus dem Hauptkatheter mittels eines im Hauptkatheter 67 angeordneten und in der Zeichnung nicht dargestellten Pushers ausgeschobene Teil der Endoprothese 60 sich bestimmungsgemäß im Körpergefäß 65 aufdehnt. Die Manschette 62 übergreift an der proximalen Stirnseite 63 den Wulst 66 und läuft dann konisch im Hauptkatheter 67 zusammen. Die konische Abschlußfläche 70 der Manschette 62 ist mit großen Seitlöchern 71 versehen, um eine nahezu ungehinderte Blutdurchströmung des behandelten Körpergefäßes 65 sicherzustellen. Hierdurch wird die Gefahr der Thrombenbildung wirksam reduziert. Im Falle eines gegenüber der Endoprothese 60 exzentrisch angeordneten Hauptkatheters 67 stehen teilweise vergrößerte konische Abschlußflächen 70 zur Verfügung, die mit entsprechend vergrößerten Seitlöchern 71 versehen werden können. Hierdurch wird die Blutperfusion weniger beeinträchtigt.

Anstelle der erfindungsgemäßen Endoprothese kann eine entsprechende Behandlung von Körpergefäßen auch mit einem entsprechend taillierten Ballonkatheter erfolgen, der ebenfalls mit Schläuchen zur Medikamentengabe oder zur Zufuhr anderer Substanzen versehen wurde. Bei einer derartigen Behandlung wird jedoch die Blutperfusion des behandelten Gefäßes für die Zeit der Behandlung unterbunden.

## Patentansprüche

1. Endoprothese, die in den Körper eines Patienten, insbesondere in ein Blutgefäß oder in eine andere Körperhöhle, mittels eines Katheters perkutan implantierbar ist , die als länglicher Hohlkörper ausgebildet und nach lagerichtiger Plazierung bei der Implantation) von einem kleinen Lumen beim Einführen auf ein der Gebrauchslage entsprechendes größeres Lumen veränderbar ist,
**dadurch gekennzeichnet,**
**daß** ein Stützkörper (11, 21, 31, 41, 51, 61) der Endoprothese (10, 20, 30, 40, 50, 60) zumindest außenseitig von einer zusammen mit dem Stützkörper (11, 21, 31, 41, 51, 61) von einem kleineren Einführ-Lumen auf ein größeres Implantations-Lumen aufweitbaren Manschette (12, 22, 32, 42, 52, 62) umschlossen ist und daß wenigstens ein mit dieser Manschette (12, 22, 32, 42, 52, 62) verbundener Zuführschlauch (14, 24, 34, 44, 54, 64) zum Zuführen von Medikamenten oder anderen Substanzen in einen sich im eingeführten Zustand zwischen der Manschette (12, 22, 32, 42, 52, 62) und der die Manschette (12, 22, 32, 42, 52, 62) umschließenden Körperhöhle ausbildenden Zwischenraum (19, 29, 39, 49, 59, 69) ausmündet.

2. Endoprothese nach Anspruch 1, **dadurch gekennzeichnet, daß** die Manschette (12, 22, 32, 42, 52, 62) aus plastisch verformbaren Material mit einer begrenzten Rückstellkraft besteht.

3. Endoprothese nach Anspruch 1, **dadurch gekennzeichnet, daß** die Manschette (12, 22, 32, 42, 52, 62) aus elastischem Material mit einer begrenzten Rückstellkraft besteht.

4. Endoprothese nach einem der Ansprüche 1 bis 3, **dadurch gekennzeichnet, daß** die Manschette (12, 22, 32, 42, 52, 62) Übermaß gegenüber dem Stützkörper (11, 21, 31, 41, 51, 61) aufweist und unter Ausbildung von Falten um diesen herumgelegt ist.

5. Endoprothese nach einem der Ansprüche 1 bis 4, **dadurch gekennzeichnet, daß** der Stützkörper (11, 21, 31, 41, 51, 61) ein proximales und ein distales Stirnende (13, 13'; 23, 23'; 33, 33'; 43, 43'; 53, 53'; 63, 63') aufweist, das jeweils gegenüber einem zwischen den Stirnenden (13, 13'; 23, 23'; 33, 33'; 43, 43'; 53, 53'; 63, 63') liegenden Mittelteil in einem aufgeweiteten Zustand der Endoprothese (10, 20, 30, 40, 50, 60) radial erweitert ist.

6. Endoprothese nach einem der Ansprüche 1 bis 5, **dadurch gekennzeichnet, daß** die Manschete (12, 22, 32, 42, 52, 62) stirnendig mit radial nach außen vorstehenden Wulsten (26, 26'; 36, 36'; 46, 46'; 56, 56'; 66, 66') versehen ist.

7. Endoprothese nach Anspruch 5, **dadurch gekennzeichnet, daß** die radial nach außen vorstehenden Wulste (26, 26') aus elastischem Material herstellbar sind und den Zwischenraum (19, 29, 39) dichtend abschließen.

8. Endoprothese nach Anspruch 7, **dadurch gekennzeichnet, daß** die radial nach außen vorstehenden Wulste (26, 26') verbreitert sind.

9. Endoprothese nach einem der Ansprüche 5 bis 8, **dadurch gekennzeichnet, daß** die Manschette (22) zwischen den Stirnenden (23, 23') mit radial nach außen weisenden vorstehenden Distanzmitteln (27) versehen ist.

10. Endoprothese nach einem der Ansprüche 5 bis 9, **dadurch gekennzeichnet, daß** ein erster Hilfskatheter (44) im Bereich des proximalen Stirnendes (43) mit der Manschette (42) und ein zweiter Hilfskatheter (44') im Bereich des Zwischenraumes (49) mit dem Stützkörper (41) verbunden ist.

11. Endoprothese nach einem der Ansprüche 1 bis 10, **dadurch gekennzeichnet, daß** die Manschette (12, 22, 32, 42, 52, 62) außenseitig mit einer Antihaftbeschichtung versehen ist.

12. Endoprothese nach einem der Ansprüche 1 bis 9, **dadurch gekennzeichnet, daß** die Manschette (32) eine schlauchartige Hohlmembran ist, die im Bereich des Mittelteiles der Endoprothese (30) mit dem Stützkörper (31) einen Manschettenhohlraum (39) ausbildet, in den ein Zuführschlauch (34) mündet.

13. Endoprothese nach Anspruch 12, **dadurch gekennzeichnet, daß** die Manschette (32) Übermaß gegenüber dem Stützkörper (31) der Endoprothese (30) aufweist, mit diesem zumindest partiell verbunden und im übrigen unter Faltenbildung um den Stützkörper (31) herumgelegt ist.

14. Endoprothese nach Anspruch 13, **dadurch gekennzeichnet, daß** die Manschette (32) von ihren Stirnenden (33, 33') aus longitudinal eingefaltet ist.

15. Endoprothese nach einem der Ansprüche 1 bis 14, **dadurch gekennzeichnet, daß** der Zuführschlauch (14, 24, 34, 44, 54, 64) mit der Manschette (12, 22, 32, 42, 52, 62) lösbar verbunden ist und daß die Manschette (12, 22, 32, 42, 52, 62) an dieser Verbindungsstelle eine Ausmündung aufweist und daß die Endoprothese (10, 20, 30, 40, 50, 60) mit einer Einrichtung versehen ist, die diese Ausmündung nach dem Entfernen des Zuführschlauchs (14, 24, 34, 44, 54, 64) verschließt.

16. Endoprothese nach Anspruch 15, **dadurch gekennzeichnet, daß** die Manschette (12, 22, 32, 42, 52, 62) aus einer sich selbstverschließenden Membran besteht, an die mittels des Katheters (47, 57, 67) ein Angiographieführungsdraht zur Punktion der Manschette (12, 22, 32, 42, 52, 62) herangeführt ist und wenigstens ein Zuführschlauch (14, 24, 34, 44, 54, 64) entlang des Führungsdrahtes derart in die Endoprothese (10, 20, 30, 40, 50, 60) eingebracht ist, daß der Zuführschlauch (14, 24, 34, 44, 54, 64) mit der durch Punktion erzeugten Ausmündung der Manschette (12, 22, 32, 42, 52, 62) verbunden ist.

17. Endoprothese nach Anspruch 15, **dadurch gekennzeichnet, daß** an die Manschette (12, 22, 32, 42, 52, 62) mittels des Katheters (47, 57, 67) ein Angiographieführungsdraht zur Punktion herangeführt ist und wenigstens ein Zuführschlauch (14, 24, 34, 44, 54, 64) entlang des Angiographieführungsdrahtes derart in die Endoprothese (10, 20, 30, 40, 50, 60) eingebracht ist, daß der Zuführschlauch (14, 24, 34, 44, 54, 64) mit der durch Punktion erzeugten Ausmündung der Manschette (12, 22, 32, 42, 52, 62) verbunden ist und daß diese Ausmündung nach Entfernen des Zuführschlauches (14, 24, 34, 54, 64) durch eine zuvor durch den Zuführschlauch (14, 24, 34, 44, 54, 64) eingebrachte polymerisierbare Substanz verschließbar ist.

18. Endoprothese nach einem der Ansprüche 1 bis 17, **dadurch gekennzeichnet, daß** die Manschette (12, 22, 32, 42, 52, 62) aus biologisch abbaubarem Material besteht.

19. Endoprothese nach einem der Ansprüche 1 bis 18 **dadurch gekennzeichnet, daß** der Stützkörper (11, 21, 31, 41, 51, 61) als durch Stricken, Häkeln oder ähnliche Formen der Maschenbildung herstellbares schlauchartiges Maschenwerk ausgebildet ist.

20. Endoprothese nach Anspruch 19, **dadurch gekennzeichnet, daß** sich vom proximalen Stirnende (13, 23, 33, 43, 53, 63) des Stützkörpers (11, 21, 31, 41, 51, 61) ein das Aufziehen des Maschenwerks ermöglichendes Endfilament forterstreckt.

21. Endoprothese nach Anspruch 20, **dadurch gekennzeichnet, daß** das Endfilament mit dem sich vom proximalen Stirnende (13, 23, 33, 43, 53, 63) forterstreckenden Zuführschlauch (14, 24, 34, 44, 54, 64) fest verbunden ist.

22. Endoprothese nach Anspruch 20, **dadurch gekennzeichnet, daß** die Manschette (12, 22, 32, 42, 52, 62) am distalen Stirnende (13', 23', 33', 43', 53', 63') der Endoprothese (10, 20, 30, 40, 50, 60) mit einem Endfilament des Stützkörpers (11, 21, 31, 41, 51, 61) verbunden ist.

23. Endoprothese nach einem der Ansprüche 5 bis 10, **dadurch gekennzeichnet, daß** der Stützkörper (11, 21, 31, 41, 51, 61) ein durch Stricken, Häkeln oder ähnliche Formen der Maschenbildung hergestelltes schlauchartiges Maschenwerk ist, das aus einem isolierten elektrisch leitenden Thermo-Memory-Draht besteht.

24. Endoprothese nach Anspruch 23, **dadurch gekennzeichnet, daß** ein Zusatzkatheter zur Infusion einer gegenüber einer normalen Bluttemperatur leicht erwärmten Flüssigkeit mit seiner Infusionsöffnung in Strömungsrichtung unmittelbar vor der proximalen Stirnseite (13, 23, 33, 43, 53, 63) der Endoprothese (10, 20, 30, 40, 50, 60) angeordnet ist.

25. Endoprothese nach Anspruch 24, **dadurch gekennzeichnet, daß** ein Stromzuleitungs- und ein Stromableitungsdraht (58 und 58') entlang des Zuführschlauches (54) angeordnet und jeweils mit dem Stützkörper (51) elektrisch leitend verbunden ist.

26. Endoprothese nach einem der vorhergehenden Ansprüche, **dadurch gekennzeichnet, daß** die implantierte Endoprothese (60) mit einem Hauptkatheter (67) derart verbunden ist, daß ein aufdehnbarer Teil der Endoprothese (60) in dem Körpergefäß (65) plaziert ist und ein anderer zusammengefalteter Teil der Endoprothese (60) im Hauptkatheter (67) verbleibt, wobei der proximale radial vorstehende Wulst (66) des ausgedehnten Teils der Endoprothese (60) von der Manschette (62) übergriffen wird und diese Manschette (62) durch einen in den Hauptkatheter (67) mündenden Konus in proximaler Richtung fortgesetzt ist und dieser Konus Seitlöcher (71), insbesondere in Form von Maschen, aufweist.

27. Endoprothese nach Anspruch 26, **dadurch gekennzeichnet, daß** innerhalb des Hauptkatheters (67) ein mit dem im Hauptkatheter (67) verbleibenden Teil der Endoprothese (60) verbundener Pusher konzentrisch angeordnet ist.

28. Endoprothese nach Anspruch 26 oder 27, **dadurch gekennzeichnet, daß** der Hauptkatheter (67) exzentrisch zu dem aufgedehnten Teil der Endoprothese (60) angeordnet ist und hierdurch eine vergrößerte proximale stirnseitige Konusfläche (70) der Manschette (62) gebildet ist, die ein entsprechend vergrößertes Seitloch (71) aufweist.

29. Endoprothese nach einem der Ansprüche 26 bis 28, **dadurch gekennzeichnet, daß** der Stützkörper (61) aus einem hoch- bis superelastischen Draht besteht.

30. Endoprothese nach einem der Ansprüche 26 bis 29, **dadurch gekennzeichnet, daß** innerhalb des mit der Endoprothese (60) verbundenen Hauptkatheters (67) ein Ballonkatheter zum Aufdehnen des in dem Körpergefäß (65) zu plazierenden Teils der Endoprothese (60) angeordnet ist.

31. Endoprothese nach einem der vorhergehenden Ansprüche, **dadurch gekennzeichnet, daß** die Manschette (12, 22, 32, 42, 52, 62) von einer zusätzlichen gel- oder schwammartigen Umhüllung umschlossen ist, die nach der Implantation der Endoprothese (10, 20, 30, 40, 50, 60) durch Flüssigkeitsaufnahme aufquillt und den Zwischenraum (19, 29, 39, 49, 59, 69) ausfüllt, wobei wenigstens ein Zuführschlauch (14, 24, 34, 44, 54, 64)in diesen Zwischenraum (19, 29, 39, 49, 59, 69) mündet.

## Claims

1. Endoprosthesis which is percutaneously implantable by means of a catheter in the body of a patient, especially in a blood vessel or in another body cavity, and which is constructed as an elongate hollow body and after positionally correct placement during the implantation is variable from a small lumen during introduction to a larger lumen corresponding with the position of use, **characterised in that** a support body (11, 21, 31, 41, 51, 61) of the endoprosthesis (10, 20, 30, 40, 50, 60) is enclosed at least at the outer side by a sleeve (12, 22, 32, 42, 52, 62) expansible together with the support body (11, 21, 31, 41, 51, 61) from a smaller introductory lumen to a larger implantation lumen and that at least one feed sleeve (14, 24, 34, 44, 54, 64), which is connected with this sleeve (12, 22, 32, 42, 52, 62), for the feed of medications or other substances opens into an intermediate space (19, 29, 39, 49, 59, 69) formed in the introduced state between the sleeve (12, 22, 32, 42, 52, 62) and the body cavity enclosing the sleeve (12, 22, 32, 42, 52, 62).

2. Endoprosthesis according to claim 1, **characterised in that** the sleeve (12, 22, 32, 42, 52, 62) consists of plastically deformable material with a limited restoring force.

3. Endoprosthesis according to claim 1, **characterised in that** the sleeve (12, 22, 32, 42, 52, 62) consists of elastic material with a limited restoring force.

4. Endoprosthesis according to one of claims 1 to 3, **characterised in that** the sleeve (12, 22, 32, 42, 52, 62) is over-dimensioned relative to the support body (11, 21, 31, 41, 51, 61) and is laid around this with formation of folds.

5. Endoprosthesis according to one of claims 1 to 4, **characterised in that** the support body (11, 21, 31, 41, 51, 61) has a proximal and a distal end (13, 13'; 23, 23'; 3, 33'; 43, 43'; 53, 53'; 63, 63') which in an expanded state of the endoprosthesis (10, 20, 30, 40, 50, 60) are each radially widened relative to a middle part disposed between the ends (13, 13'; 23, 23'; 3, 33'; 43, 43'; 53, 53'; 63, 63').

6. Endoprosthesis according to one of claims 1 to 5, **characterised in that** the sleeve (12, 22, 32, 42, 52, 62) is provided at the ends with radially outwardly protruding beads (26, 26'; 36, 36'; 46, 46'; 56, 56'; 66, 66').

7. Endoprosthesis according to claim 5, **characterised in that** the radially outwardly protruding beads (26, 26') are producible from elastic material and sealingly dose off the intermediate space (19, 29, 39).

8. Endoprosthesis according to claim 7, **characterised in that** the radially outwardly protruding beads (26, 26') are widened.

9. Endoprosthesis according to one of claims 5 to 8, **characterised in that** the sleeve (22) is provided between the ends (23, 23') with radially outwardly facing protruding spacers (27).

10. Endoprosthesis according to one of dams 5 to 9, **characterised in that** a first auxiliary catheter (44) is connected with the sleeve (42) in the region of the proximal end (43) and a second auxiliary catheter (44') is connected with the support body (41) in the region of the intermediate space (49).

11. Endoprosthesis according to one of claims 1 to 10, **characterised in that** the sleeve (12, 22, 32, 42, 52, 62) is provided at the outer side with an anti-adhesion coating.

12. Endoprosthesis according to one of claims 1 to 9, **characterised in that** the sleeve (32) is a hose-like hollow membrane which in the region of the middle part of the endoprosthesis (30) forms together with the support body (31) a sleeve cavity (39) which opens into a feed hose (34).

13. Endoprosthesis according to claim 12, **characterised in that** the sleeve (32) is over-dimensioned relative to the support body (31) of the endoprosthesis (30), is connected at least partially therewith and for the remainder is laid around the support body (31) with formation of folds.

14. Endoprosthesis according to claim 13, **characterised in that** the sleeve (32) is longitudinally folded-in going out from the ends (33, 33') thereof.

15. Endoprosthesis according to one of claims 1 to 14, **characterised in that** the feed hose (14, 24, 34, 44, 54, 64) is detachably connected with the sleeve (12, 22, 32, 42, 52, 62) and that the sleeve (12, 22, 32, 42, 52, 62) has a mouth at this connecting point and that the endoprosthesis (10, 20, 30, 40, 50, 60) is provided with a device which closes this mouth after removal of the feed hose (14, 24, 34, 44, 54, 64).

16. Endoprosthesis according to claim 13, **characterised in that** the sleeve (12, 22, 32, 42, 52, 62) consists of a self-dosing membrane to which an angiography guide wire for punction of the sleeve (12, 22, 32, 42, 52, 62) is guided by means of the catheter (47, 57, 67) and at least one feed hose (14, 24, 34, 44, 54, 64) is introduced into the endoprosthesis (10, 20, 30, 40, 50, 60) along the guide wire in such a manner that the feed hose (14, 24, 34, 44, 54, 64) is connected with the mouth, which is produced by puncturing, of the sleeve (12, 22, 32, 42, 52, 62).

17. Endoprosthesis according to claim 15, **characterised in that** an angiography guide wire for punction is led to the sleeve (12, 22, 32, 42, 52, 62) by means of the catheter (47, 57, 67) and at least one feed hose (14, 24, 34, 44, 54, 64) is introduced into the endoprosthesis (10, 20, 30, 40, 50, 60) along the angiography guide wire in such manner that the feed hose (14, 24, 34, 44, 54, 64) is connected with the mouth, which is produced by puncturing, of the sleeve (12, 22, 32, 42, 52, 62) and that this mouth after removal of the feed hose (14, 24, 34, 44, 54, 64) is closable by a polymerisable substance previously introduced through the feed hose (14, 24, 34, 44, 54, 64).

18. Endoprosthesis according to one of claims 1 to 17, **characterised in that** the sleeve (12, 22, 32, 42, 52, 62) consists of biologically degradable material.

19. Endoprosthesis according to one of claims 1 to 18, **characterised in that** the support body (11, 21, 31, 41, 51, 61) is formed as a hose-like meshwork producible by knitting, crocheting or similar forming of the stitch formation.

20. Endoprosthesis according to claim 19, **characterised in that** an end filament enabling drawing up of the meshwork continues on from the proximal end (13, 23, 33, 43, 53, 63) of the support body (11, 21, 31, 41, 51, 61).

21. Endoprosthesis according to claim 20, **characterised in that** the end filament is fixedly connected with the feed hose (14, 24, 34, 44, 54, 64) extending on from the proximal end (13, 23, 33, 43, 53, 63).

22. Endoprosthesis according to claim 20, **characterised in that** the sleeve (12, 22, 32, 42, 52, 62) is connected with an end filament of the support body (11, 21, 31, 41, 51, 61) at the distal end (13', 23', 33', 43', 53', 63') of the endoprosthesis (10, 20, 30, 40, 50, 60).

23. Endoprosthesis according to one of claims 5 to 10, **characterised in that** the support body (11, 21, 31, 41, 51, 61) is a hose-like meshwork produced by knitting, crocheting or similar forming of the stitch formation and which consists of an insulated, electrically conductive thermo-memory wire.

24. Endoprosthesis according to claim 23, **characterised in that** an auxiliary catheter for infusion of a liquid, which is easily heated relative to normal blood temperature, is arranged by its infusion opening in flow direction directly in front of the proximal end (13, 23, 33, 43, 53, 63) of the endoprosthesis (10, 20, 30, 40, 50, 60).

25. Endoprosthesis according to claim 24, **characterised in that** a current feed wire (58) and a current discharge wire (58') are arranged along the feed hose (54) and each electrically conductively connected with the support body (51).

26. Endoprosthesis according to one of the preceding claims, **characterised in that** the implanted endoprosthesis (60) is connected with a main catheter (67) in such a manner that an extensible part of the endoprosthesis (60) is placed in the body vessel (65) and another, folded-together part of the endoprosthesis (60) remains in the main catheter (67), wherein the proximal radially protruding bead (66) of the expanded part of the endoprosthesis (60) is engaged over by the sleeve (62) and this sleeve (62) is continued by a cone, which opens into the main catheter (67), in proximal direction and this cone has lateral holes (71), particularly in the form of meshes.

27. Endoprosthesis according to claim 26, **characterised in that** a pusher connected with the part of the endoprosthesis (60) remaining in the main catheter (67) is concentrically arranged within the main catheter (67).

28. Endoprosthesis according to claim 26 or 27, **characterised in that** the main catheter (67) is arranged concentrically with the expanded part of the endoprosthesis (60) and thereby an enlarged proximal end cone surface (70) of the sleeve (62) is formed and has a correspondingly enlarged lateral hole (71).

29. Endoprosthesis according to one of claims 26 to 28, **characterised in that** the support body (61) consists of a highly elastic to super-elastic wire.

30. Endoprosthesis according to one of claims 26 to 29, **characterised in that** a balloon catheter for extending the part of the endoprosthesis (60) to be placed in the body vessel (65) is arranged within the main catheter (67) connected with the endoprosthesis (60).

31. Endoprosthesis according to one of the preceding claims, **characterised in that** sleeve (12, 22, 32, 42, 52, 62) is enclosed by an additional gel-like or sponge-like casing which after implantation of the endoprosthesis (10, 20, 30, 40, 50, 60) swells up by liquid absorption and fills out the intermediate space (19, 29, 39, 49, 59, 69), wherein at least one feed hose (14, 24, 34, 44, 54, 64) opens into this intermediate space (19, 29, 39, 49, 59, 69).

## Revendications

1. Endoprothèse, qui peut être implantée par voie percutanée dans le corps d'un patient, en particulier dans un vaisseau sanguin ou dans une autre cavité du corps, au moyen d'un cathéter, qui est conçue comme un corps creux allongé et peut être modifié après un emplacement correct lors de l'implantation d'un petit lumen lors de l'introduction en un lumen plus grand approprié à la position d'utilisation,
**caractérisée en ce que**
un corps de support (11, 21, 31, 41, 51, 61) de l'endoprothèse (10, 20, 30, 40, 50, 60) est entouré au moins côté extérieur par une manchette (12, 22, 32, 42, 52, 62) pouvant être élargie en même temps que le corps de support (11, 21, 31, 41, 51, 61) d'un lumen d'implantation plus petit en un lumen d'implantation plus grand et **en ce qu'**au moins un flexible d'alimentation (14, 24, 34, 44, 54, 64) relié à cette manchette (12, 22, 32, 42, 52, 62) pour l'arrivée des médicaments ou autres substances débouche dans un espace intermédiaire (19, 29, 39, 49, 59, 69) se formant dans l'état introduit entre la manchette (12, 22, 32, 42, 52, 62) et la cavité de corps entourant la manchette (12, 22, 32, 42, 52, 62).

2. Endoprothèse selon la revendication 1, **caractérisée en ce que** la manchette (12, 22, 32, 42, 52, 62) est à base de matériau plastiquement déformable avec une force de rappel limitée.

3. Endoprothèse selon la revendication 1, **caractérisée en ce que** la manchette (12, 22, 32, 42, 52, 62) est à base de matériau élastique avec une force de rappel limitée.

4. Endoprothèse selon l'une quelconque des revendications 1 à 3, **caractérisée en ce que** la manchette (12, 22, 32, 42, 52, 62) présente une surcote par rapport au corps d'appui (11, 21, 31, 41, 51, 61) et est posée autour de ce corps en formant des plis.

5. Endoprothèse selon l'une quelconque des revendications 1à 4, **caractérisée en ce que** le corps de support (11, 21, 31, 41, 51, 61) est une extrémité avant proximale et une extrémité avant distale (13, 13' ; 23, 23' ; 33, 33' ; 43, 43' ; 53, 53' ; 63, 63'), qui est élargie dans le sens radial à chaque fois par rapport à une partie centrale disposée entre les extrémités avant (13, 13' ; 23, 23' ; 33, 33' ; 43, 43' ; 53, 53' ; 63, 63') dans un état élargi de l'endoprothèse (10, 20, 30, 40, 50, 60).

6. Endoprothèse selon l'une quelconque des revendications 1 à 5, **caractérisée en ce que** la manchette (12, 22, 32, 42, 52, 62) est pourvue sur l'extrémité avant des renflements (26, 26' ; 36, 36' ; 46, 46' ; 56, 56' ; 66, 66') avançant radialement vers l'extérieur.

7. élastique selon la revendication 5, **caractérisée en ce que** les renflements (26, 26') avançant radialement vers l'extérieur peuvent être fabriqués à base de matériau élastique et ferment l'espace intermédiaire (19, 29, 39) en le rendant étanche.

8. Endoprothèse selon la revendication 7, **caractérisée en ce que** les renflements (26, 26') avançant radialement vers l'extérieur sont élargis.

9. Endoprothèse selon l'une quelconque des revendications 5 à 8, **caractérisée en ce que** la manchette (22) est pourvue entre les extrémités avant (23, 23') de moyens d'écartement saillants dirigés radialement vers l'extérieur.

10. Endoprothèse selon l'une quelconque des revendications 5 à 9, **caractérisée en ce qu'**un premier cathéter auxiliaire (44) est relié à la manchette (42) dans la zone de l'extrémité frontale (43) proximale et un second cathéter auxiliaire (44') est relié au corps de support (41) dans la zone de l'espace intermédiaire (49).

11. Endoprothèse selon l'une quelconque des revendications 1 à 10, **caractérisée en ce que** la manchette (12, 22, 32, 42, 52, 62) est pourvue côté extérieur d'un revêtement antiadhésif.

12. Endoprothèse selon l'une quelconque des revendications 1 à 9, **caractérisée en ce que** la manchette (32) est une membrane creuse en forme de flexible, qui forme dans la zone de la partie centrale de l'endoprothèse (30) avec le corps de support (31) une cavité de manchette (39), dans laquelle débouche un flexible d'alimentation (34).

13. Endoprothèse selon la revendication 12, **caractérisée en ce que** la manchette (32) présente une surcote par rapport au corps de support (31) de l'endoprothèse (30), est reliée au moins partiellement à ce corps et est posée d'autre part autour du corps de support (31) en formant des plis.

14. Endoprothèse selon la revendication 13, **caractérisée en ce que** la manchette (32) est pliée à l'intérieur dans le sens longitudinal à partir de ses extrémités avant (33, 33').

15. Endoprothèse selon l'une quelconque des revendications 1 à 14, **caractérisée en ce que** le flexible d'alimentation (14, 24, 34, 44, 54, 64) est relié de façon amovible à la manchette (12, 22, 32, 42, 52, 62) et **en ce que** la manchette (12, 22, 32, 42, 52, 62) présente un orifice de sortie en ce point de liaison et **en ce que** l'endoprothèse (10, 20, 30, 40, 50, 60) est pourvue d'un système qui ferme cet orifice de sortie après l'enlèvement du flexible d'alimentation (14, 24, 34, 44, 54, 64).

16. Endoprothèse selon la revendication 15, **caractérisée en ce que** la manchette (12, 22, 32, 42, 52, 62) comprend une membrane fermant automatiquement de laquelle un fil de guidage d'angiographie est approché au moyen du cathéter (47, 57, 67) pour la ponction de la manchette (12, 22, 32, 42, 52, 62) et au moins un flexible d'arrivée (14, 24, 34, 44, 54, 64) est introduit le long du fil de guidage dans l'endoprothèse (10, 20, 30, 40, 50, 60) de telle sorte que le flexible d'alimentation (14, 24, 34, 44, 54, 64) est relié à l'orifice de sortie, généré par la ponction, de la manchette (12, 22, 32, 42, 52, 62).

17. Endoprothèse selon la revendication 15, **caractérisée en ce qu'**un fil de guidage d'angiographie est approché de la manchette (12, 22, 32, 42, 52, 62) au moyen du cathéter (47, 57, 67) pour la ponction et qu'au moins un flexible d'alimentation (14, 24, 34, 44, 54, 64) est introduit le long du fil de guidage d'angiographie dans l'endoprothèse (10, 20, 30, 40, 50, 60) de telle sorte que le flexible d'alimentation (14, 24, 34, 44, 54, 64) est relié à l'orifice de sortie généré par ponction de la manchette (12, 22, 32, 42, 52, 62) et **en ce que** cet orifice de sortie peut être fermé après l'enlèvement du flexible d'alimentation (14, 24, 34, 44, 54, 64) par une substance polymérisable introduite auparavant par le flexible d'alimentation (14, 24, 34, 44, 54, 64).

18. Endoprothèse selon l'une quelconque des revendications 1 à 17, **caractérisée en ce que** la manchette (12, 22, 32, 42, 52, 62) est à base de matériau biodégradable.

19. Endoprothèse selon l'une quelconque des revendications 1 à 18, **caractérisée en ce que** le corps de support (11, 21, 31, 41, 51, 61) est conçu comme un réseau de mailles de type flexible pouvant être fabriqué par tricotage, crochet ou des formes similaires de formation de mailles.

20. Endoprothèse selon la revendication 19, **caractérisée en ce qu'**un filament final permettant le montage du réseau de mailles se poursuit à partir de l'extrémité avant (13, 23, 33, 43, 53, 63) proximale du corps de support (11, 21, 31, 41, 51, 61).

21. Endoprothèse selon la revendication 20, **caractérisée en ce que** le filament final est relié de façon fixe au flexible d'alimentation (14, 24, 34, 44, 54, 64) s'étendant à partir de l'extrémité avant (13, 23, 33, 43, 53, 63) proximale.

22. Endoprothèse selon la revendication 20, **caractérisée en ce que** la manchette (12, 22, 32, 42, 52, 62) est reliée sur l'extrémité avant (13', 23', 33', 43', 53', 63') distale de l'endoprothèse (10, 20, 30, 40, 50, 60) à un filament final du corps de support (11, 21, 31, 41, 51, 61).

23. Endoprothèse selon l'une quelconque des revendications 5 à 10, **caractérisée en ce que** le corps de support (11, 21, 31, 41, 51, 61) est un réseau de mailles de type flexible formé par tricotage, crochet ou formes similaires de formation de mailles, qui est à base d'un fil "thermo-memory" électroconducteur et isolé.

24. Endoprothèse selon la revendication 23, **caractérisée en ce qu'**un cathéter supplémentaire pour la perfusion d'un liquide légèrement réchauffé par rapport à une température de sang normal est disposé avec son ouverture de perfusion dans le sens d'écoulement directement avant l'extrémité avant (13, 23, 33, 43, 53, 63) proximale de l'endoprothèse (10, 20, 30, 40, 50, 60).

25. Endoprothèse selon la revendication 24, **caractérisée en ce qu'**un fil d'arrivée de courant et un fil de départ de courant (58, 58') sont disposés le long du flexible d'alimentation (54) et reliés de façon électroconductrice respectivement au corps de support (51).

26. Endoprothèse selon l'une quelconque des revendications précédentes, **caractérisée en ce que** l'endoprothèse (60) implantée est reliée à un cathéter principal (67) de telle sorte qu'une partie étirable de l'endoprothèse (60) est placée dans le vaisseau du corps (65) et une autre partie repliée de l'endoprothèse (60) reste dans le cathéter principal (67), le renflement (66) proximal et débordant radialement de la partie étendue de l'endoprothèse (60) est recouvert par la manchette (62) et cette manchette (62) est poursuivie dans le sens proximal par un cône débouchant dans le cathéter principal (67) et ce cône présente des trous latéraux (71),en particulier sous la forme de mailles.

27. Endoprothèse selon la revendication 26, **caractérisée en ce qu'**à l'intérieur du cathéter principal (67) est disposé de façon concentrique un pusher relié à la partie de l'endoprothès (60) restant dans le cathéter principal (67).

28. Endoprothèse selon la revendication 26 ou 27, **caractérisée en ce que** le cathéter principal (67) est disposé de façon excentrique par rapport à la partie étendue de l'endoprothèse (60) et de ce fait une surface conique (70) côté avant, proximale et agrandie de la manchette (62) est formée, laquelle présente un trou latéral (71) agrandi en conséquence.

29. Endoprothèse selon l'une quelconque des revendications 26 à 28, **caractérisée en ce que** le corps de support (61) comprend un fil très élastique à super élastique.

30. Endoprothèse selon l'une quelconque des revendications 26 à 29, **caractérisée en ce qu'**un cathéter à ballonnet pour l'allongement de la partie de l'endoprothèse (60) à placer dans le vaisseau du corps (65) est disposé à l'intérieur du cathéter principal (67) relié à l'endoprothèse (60).

31. Endoprothèse selon l'une quelconque des revendications précédentes, **caractérisée en ce que** la manchette (12, 22, 32, 42, 52, 62) est entourée d'une enveloppe supplémentaire du type gel ou éponge, qui gonfle après l'implantation de l'endoprothèse (10, 20, 30, 40, 50, 60) du fait de l'absorption de liquide et remplit l'interstice (19, 29, 39, 49, 59, 69), au moins un flexible d'arrivée (14, 24, 34, 44, 54, 64) débouchant dans cet espacement (19, 29, 39, 49, 59, 69).
